# EUROPEAN PATENT APPLICATION

(11) **EP 3 352 173 A1**
(43) Date of publication of application: **25.07.2018**
(21) Application number: 17209100.1
(22) Date of filing: 01.03.2013
(51) Int. Cl.: G16H 20/30, A61H 23/04

(54) **SEQUENTIAL COMPRESSION THERAPY COMPLIANCE MONITORING SYSTEMS AND METHODS**

(30) Priority: 02.03.2012 US 201213410502
(62) Divisional of application: 13755188.3
(71) Applicant: Hill-Rom Services, Inc., Batesville, IN 47006 (US)
(72) Inventor: HORNBACH, David, W, Batesville, IN 47006 (US); RIBBLE, David, L, Batesville, IN 47006 (US); O'KEEFE, Christopher, R, Batesville, IN 47006 (US); RECEVEUR, Timothy, J, Batesville, IN 47006 (US); SKRIPPS, Thomas, K, Batesville, IN 47006 (US)
(74) Representative: Loustalan, Paul William

(57) **Abstract**

A system to determine usage and monitor compliance of a Sequential Compression Therapy (SCT) device is disclosed. The system is configured to track the duration, amplitude and frequency of application of sequential compression therapy and communicate this information over a communication network. The system further includes sensing capability incorporated in the compression sleeve to monitor physiological signals. The system is configured to trigger an alarm if either the duration of the sequential compression therapy or the physiological signal does not meet a predetermined threshold.

## Description

### BACKGROUND

Sequential compression therapy (SCT) is often used for prevention of deep venous thrombosis (DVT). Traditionally monitoring compliance and the utilization of sequential compression therapy by a patient are accomplished by a caregiver. While various systems have been developed for monitoring sequential compression therapy, there is still room for improvement. Thus, a need persists for further contribution in this area of technology.

### BRIEF SUMMARY

The present disclosure includes one or more of the features recited in the appended claims and/or the following features which, alone or in any combination, may comprise patentable subject matter.

One embodiment of the system to track usage of a compression sleeve comprises a compression sleeve, a fluid supply operable to inflate the compression sleeve, a processor configured to control the operation of the fluid supply and a memory device configured to record duration of the inflation of the compression sleeve.

Another embodiment of the system to monitor compliance of a compression sleeve usage comprises a memory device configured to record usage of a compression sleeve, a processor configured to compare the usage with a predetermined threshold.

One embodiment of the method to monitor compliance of a compression sleeve usage comprises determining usage of a compression sleeve, comparing usage of a compression sleeve with a predetermined threshold and determining compliance by comparing usage of a compression sleeve with the predetermined threshold.

Another embodiment of a system to monitor compliance of a compression sleeve usage comprises a compression sleeve and a first processor mounted on the compression sleeve configured to determine duration of inflation of the compression sleeve and communicate with a second processor mounted on a person support apparatus.

### BRIEF DESCRIPTION OF DRAWINGS

The accompanying drawings incorporated in and forming a part of the specification illustrate several aspects of the claimed subject matter and, together with the description, serve to explain the principles of the claimed subject matter. In the drawings:
FIG. 1 is a perspective view of a system to track usage of a compression sleeve integrated with a hospital bed, constructed according to principles of one or more of the teachings herein;
FIG. 2 is a perspective view of a system to track usage of a compression sleeve integrated with a fracture table, constructed according to principles of one or more of the teachings herein;
FIG. 3 is a side view of a compression sleeve mounted on a patient's leg, constructed according to principles of one or more of the teachings herein;
FIG. 4 is a block diagram of a system to monitor compliance of a compression sleeve, integrated with a hospital bed, constructed according to principles of one or more of the teachings herein;
FIG. 5 is a block diagram of another system to monitor compliance of a compression sleeve, integrated with a hospital bed, constructed according to principles of one or more of the teachings herein;

### DETAILED DESCRIPTION OF ILLUSTRATIVE EMBODIMENTS

The embodiments of the claimed subject mater and the various features and advantageous details thereof are explained more fully with reference to the non-limiting embodiments and examples that are described and/or illustrated in the accompanying drawings and detailed in the following description. It should be noted that the features illustrated in the drawings are not necessarily drawn to scale, and features of one embodiment may be employed with other embodiments as the skilled artisan would recognize, even if not explicitly stated herein. Descriptions of well-known components and processing techniques may be briefly mentioned or omitted so as to not unnecessarily obscure the embodiments of the claimed subject matter described. The examples used herein are intended merely to facilitate an understanding of ways in which the claimed subject matter may be practiced and to further enable those of skill in the art to practice the embodiments of the claimed subject matter described herein. Accordingly, the examples and embodiments herein are merely illustrative and should not be construed as limiting the scope of the claimed subject matter, which is defined solely by the appended claims and applicable law. Moreover, it is noted that like reference numerals represent similar parts throughout the several views of the drawings.

It is understood that the subject matter claimed is not limited to the particular methodology, protocols, devices, apparatus, materials, applications, etc., described herein, as these may vary. It is also to be understood that the terminology used herein is used for the purpose of describing particular embodiments only, and is not intended to limit the scope of the claimed subject matter.

Unless defined otherwise, all technical and scientific terms used herein have the same meanings as commonly understood by one of ordinary skill in the art.

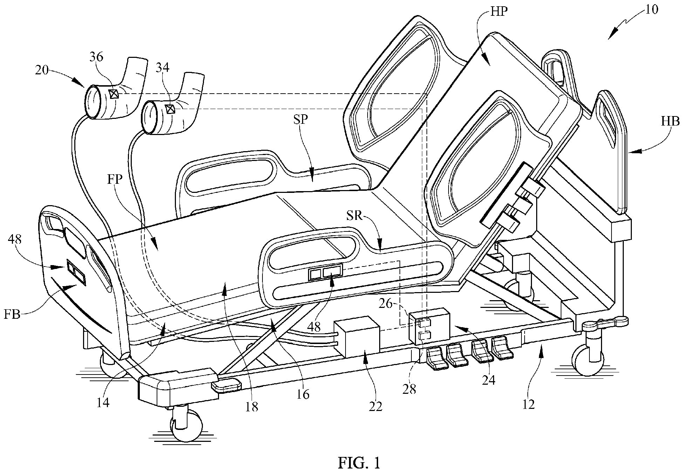

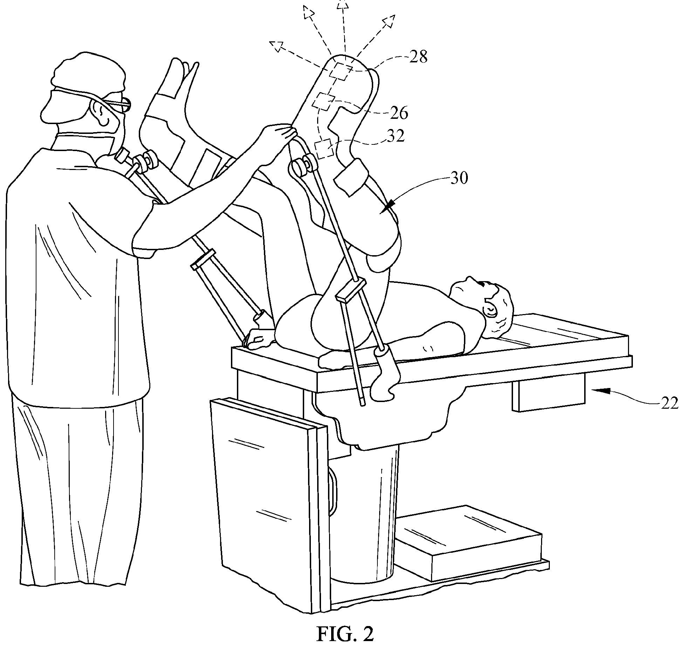

A person support apparatus 10 according to one illustrative embodiment of the current disclosure is shown in FIG. 1. In the illustrative embodiment as described in FIG. 1, the person support apparatus 10 is a hospital bed. The person support apparatus 10 includes a lower frame or base 12, a plurality of supports 16 coupled with the lower frame 12, and an upper frame 14 supported by the plurality of supports above the lower frame 12. The upper frame 14 is configured to variably elevate with respect to the lower frame 12. A person support surface 18 is mounted on at least a portion of the person support apparatus 10. The person support surface 18 has a head support section HP, a seat support section SP and a foot support section FP as shown in FIG. 1. The person support apparatus 10 may be a stretcher or an operating table in another embodiment as described in FIG 2. FIG.1 illustrates a system to track usage of a compression sleeve comprising a fluid supply 22 mounted on the person support apparatus 10 and fluidly connected to the compression sleeve 20 using any one of a multitude of connection mechanisms, including quick disconnect type connectors. Compression sleeve 20 may be of any form, including but not limited to cuffs, boots and / or a wrap and may be customized to fit any body part. In one embodiment, the fluid supply 22 is removably mounted on the foot board FB, head board HB or the side rails SR. In another embodiment, the fluid supply 22 may be mounted on the compression sleeve 22. The fluid supply 22 may be used for other functions of the person support apparatus 10, including but not limited to inflation and / or deflation of a mattress. In another embodiment, the system to track usage of a compression sleeve 20 comprises a dedicated fluid supply 22. The fluid supply 22 provides fluid to the compression sleeve 20 during inflation and vents the fluid from the compression sleeve 20 during deflation of the compression sleeve 20 in one embodiment. The fluid supply unit may be pneumatic or hydraulic in nature and may comprise a pump, blower, fan and/ or a compressor. Therefore, the compression sleeve may be inflated with air, water, oil or any other fluid. A controller 24 comprises a processor 26 in communication with the fluid supply 22 and provides a control signal to the fluid supply 22 to inflate or deflate the compression sleeve 20. The processor 26 also communicates with a pressure sensor 34 mounted on the compression sleeve 20 to allow measurement of the pressure inside the inflated compression sleeve 20 and / or the pressure applied on a patient's extremity to which the compression sleeve 20 is mounted. Alternatively, back pressure and / or electrical characteristics such as current and voltage changes of the prime mover of the fluid supply 22 may be used to determine the pressure in the compression sleeve in another embodiment. An electrical stimulation device 36 is mounted in the compression sleeve 20 to provide appropriate therapy to the patient. The electrical stimulation device 36 may be of any type, including a Transcutaneous Nerve Stimulation (TENS) type for blocking pain and / or an Electro Muscle Stimulation (EMS) device for stimulating muscles. The electrical stimulation device 36 is in communication with and receives control signals from the processor 26 to initiate or terminate therapy. In one embodiment, the electrical stimulation device 36 receives power from the controller 24 while in another embodiment the electrical stimulation device 36 has a dedicated power source such as a battery pack. The controller 24 is in communication with a Graphical User Interface (GUI) 48 to display alarms and other messages. The GUI 48 is integral to the person support apparatus 10 in one embodiment and is used to control other functions of the person support apparatus 10. The GUI 48 also allows for user inputs and commands to set thresholds for the amplitude, frequency and / or time duration of SCT and / or electrical stimulation therapy. These thresholds may be in the form of an upper bound (should not exceed), lower bound (should be greater than) and / or a range. The processor 26 uses these thresholds to determine compliance with prescribed therapy. In one embodiment, the duration of cumulative therapy is tracked by the processor 26, by storing, in a memory device 28, data related to inflation and deflation of the compression sleeve 20 and / or operation of the electrical stimulation device. The memory device 28 may be of any type, including volatile and non-volatile types. If the cumulative duration of therapy does not meet the criteria established earlier, an alarm will be displayed on the GUI 48. Display of other performance characteristics of the therapy on the GUI 48 is well within the scope of this disclosure, including, but not limited to cumulative duration of therapy, duration of therapy of current session, average amplitude of therapy which in one embodiment may be the pressure and / or the electrical stimulation applied and frequency of therapy. The GUI 48 also allows a user to clear previously stored data in the memory device 28 and / or initiate therapy. In another embodiment, the system to track usage of a compression sleeve includes a dedicated GUI 48, which may be in the form of a pendant.

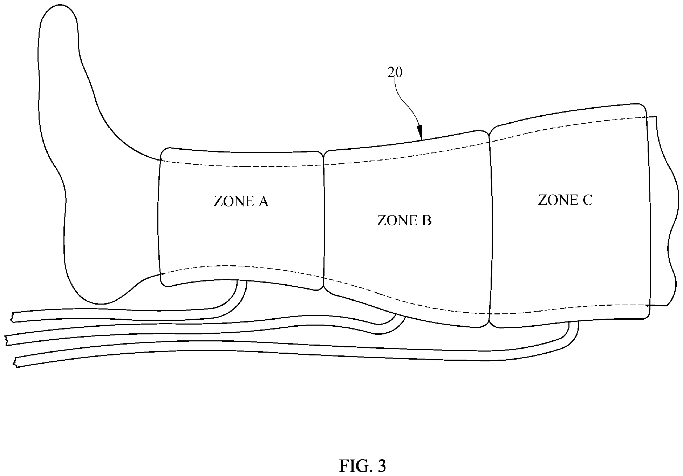

The illustrative embodiment as described in FIG.2 shows a compression sleeve 20 mounted in a stirrup 30 used in conjunction with an operating table. In the embodiment described in FIG. 2, the processor 26 and the memory device 28 are mounted on the compression sleeve 20. The compression sleeve 20 is configured to couple to a fluid supply 22 mounted on the operating table using any type of connection mechanism, including a quick disconnect mechanism. In another embodiment, the fluid supply 22 may be mounted on the stirrup 30 or the compression sleeve 22. The processor 26 and / or the memory device 28 wirelessly communicate with a communication network 40 (FIG.3). The communication network 40 is in turn connected to a GUI 48, not shown in FIG. 2 and the GUI 48 allows for inputting commands, display of messages and / or for communicating alarms received from the processor 26 and / or the memory device 28. The illustrative embodiment as shown in FIG. 2 also describes a physiological sensor 32 mounted on the compression sleeve 22 in communication with the processor 26. It is well within the scope of the disclosure to include more than one physiological sensor 32 in the system. Physiological sensor 32 may be of any type and communicates with the processor 26 by way of an electrical signal indicative of at least one physiological parameter, including but not limited to temperature, blood pressure, heart rate and electrical activity. Processor 26 monitors the signals from the physiological sensor 32 and is configured to communicate an alarm to the GUI 48 in response to any of the physiological parameters not complying with a predetermined rule or threshold. The GUI is also configured to display data obtained from physiological sensors 32 via processor 26. The illustrative embodiment as described in FIG.2 allows for the same compression sleeve to be used in post-operative care as was used during an operation. This transfer from an operating room to post-operative care often involves changing rooms and / or person support apparatus 10. Since the system as described in FIG. 2 allows for mounting the processor 26 and memory device 28 on the compression sleeve 22, a continuous stream of data is available and a cumulative usage of the compression sleeve may be determined. The processor 26 and / or memory device 28 are configured to communicate with other controllers comprising processors and / or GUIs which in one embodiment may be remote. The processor 26 and / or the memory device 28 may communicate with a controller comprising a processor and / or GUI 48 integral to a patient support apparatus 10 via a wireless or wired connection based on the physical distance between the two devices. Thus, as the patient is moved from the operating room to a post-operative room, the processor 26 and / or the memory device 28 automatically connects with the controller of the person support apparatus 10 in the post-operative room. As described earlier, a fluid supply 22 mounted on the compression sleeve 20 allows for continued therapy during patient transfer.

The illustrative embodiment as described in FIG. 3 shows one embodiment of a compression sleeve 20 for use in the system to track usage of a compression sleeve. FIG. 3 shows the compression sleeve 20 used for sequential compression therapy of the patient's calf region. As shown in FIG. 3, the compression sleeve 20 has more than one zone in this embodiment, although a single pressurized zone is well within the scope of the current disclosure. The sleeve as described in FIG. 3 has fluid supply lines connected to each zone, although a single supply line all the zones may be used in another embodiment.

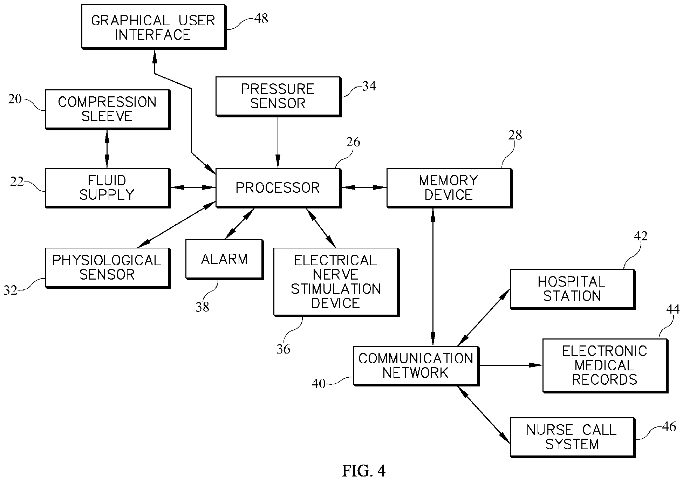

FIG. 4 shows a block diagram of one embodiment of the system to track usage of a compression sleeve. The compression sleeve 20 is supplied fluid by a fluid supply 22, which in turn receives a control signal from the processor 26. At least one pressure sensor 34 mounted in the compression sleeve is in electrical communication with the processor 26. At least one electrical stimulation device 36 is also in communication with the processor 26. The processor 26 communicates with a memory device 28 and records parameters related to the sequential compression therapy, including, but not limited to any one or combination of the cumulative duration of therapy, duration of therapy for an individual session, amplitude and frequency of pressure applied. The processor 26 also conveys data corresponding to the signals received from at least one physiological sensor 32 and the electrical stimulation device 36 to the memory device 28. The processor 26 is also in communication with an alarm 38, which may be any combination of an audio, visual or tactile output. The processor 26 is configured to enable the alarm 38 based on the thresholds for sequential compression therapy, signals from the electrical stimulation device 36 or the physiological sensor 32. The processor 26 is in communication with a graphical user interface (GUI) 48 which allows for input of control parameters by a user and for display of system messages. Alarm 38 may be triggered and in another embodiment a timer may be activated for the alarm to trigger after a predetermined time period.

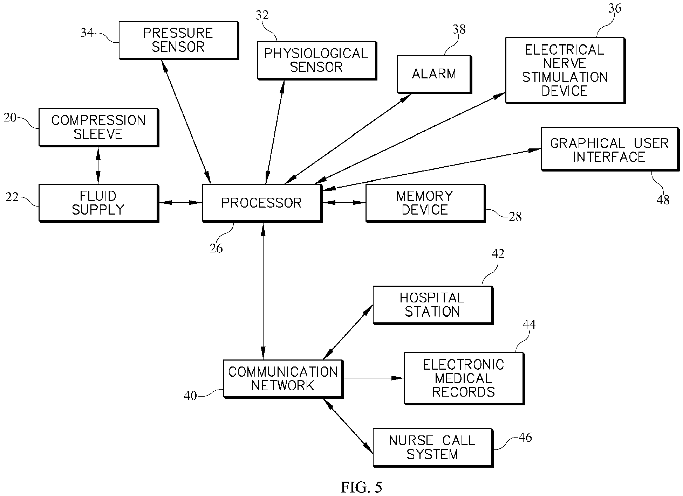

In the illustrative embodiment shown in FIG. 4, the memory device 28 is connected to a communication network 40. The communication network 40 may be of any type, including but not limited to Wide Area Network (WAN), Local Area Network (LAN), Virtual Private Network (VPN), telephone lines, optical communications, internet communications or telex. The communication network 40 is in communication with at least one Electronic Medical Record (EMR) 44 or another healthcare information database, a hospital communication station 42 and a nurse call system 46. The communication station 42 is a centralized location wherein one or more patients may be monitored by a caregiver. In one embodiment the communication station 42 may be stationary, while in another embodiment, it may be a mobile unit to accommodate prompt deployment. In the illustrative embodiment shown in FIG. 5, the processor 26 is connected to a communication network 40. The communication network 40 may be of any type, including but not limited to Wide Area Network (WAN), Local Area Network (LAN), Virtual Private Network (VPN), telephone lines, optical communications, internet communications or telex. The communication network 40 is in communication with at least one Electronic Medical Record (EMR) 44 or another healthcare information database, a hospital communication station 42 and a nurse call system 46. The communication station 42 is a centralized location wherein one or more patients may be monitored by a caregiver. In one embodiment the communication station 42 may be stationary, while in another embodiment, it may be a mobile unit to accommodate prompt deployment.

The use of the terms "a" and "an" and "the" and similar referents in the context of describing the subject matter (particularly in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. Furthermore, the foregoing description is for the purpose of illustration only, and not for the purpose of limitation, as the scope of protection sought is defined by the claims as set forth hereinafter together with any equivalents thereof entitled to. The use of any and all examples, or exemplary language (e.g., "such as") provided herein, is intended merely to better illustrate the subject matter and does not pose a limitation on the scope of the subject matter unless otherwise claimed. The use of the term "based on" and other like phrases indicating a condition for bringing about a result, both in the claims and in the written description, is not intended to foreclose any other conditions that bring about that result. No language in the specification should be construed as indicating any non-claimed element as essential to the practice of the invention as claimed.

Preferred embodiments are described herein, including the best mode known to the inventor for carrying out the claimed subject matter. Of course, variations of those preferred embodiments will become apparent to those of ordinary skill in the art upon reading the foregoing description. The inventor expects skilled artisans to employ such variations as appropriate, and the inventor intends for the claimed subject matter to be practiced otherwise than as specifically described herein. Accordingly, this claimed subject matter includes all modifications and equivalents of the subject matter recited in the claims appended hereto as permitted by applicable law. Moreover, any combination of the above-described elements in all possible variations thereof is encompassed unless otherwise indicated herein or otherwise clearly contradicted by context.

The disclosures of any references and publications cited above are expressly incorporated by reference in their entireties to the same extent as if each were incorporated by reference individually

Further features of the present disclosure are provided in the following list of numbered clauses, and dependent clauses:
Clause 1. A system to track usage of a compression sleeve comprising:
   a fluid supply operable to inflate said compression sleeve;
   a processor coupled to a person support apparatus, said processor is configured to control operation of said fluid supply; and
   a memory device configured to record duration of said inflation of said compression sleeve.
Clause 2. The system of clause 1 wherein said memory device is configured to communicate data with a communication network.
Clause 3. The system of clause 2 wherein said communication network is configured to communicate with an electronic medical record.
Clause 4. The system of clause 2 or 3 wherein said communication network is configured to communicate with a nurse call system.
Clause 5. The system of clause 2, 3 or 4 wherein said communication network is configured to communicate with a hospital station.
Clause 6. The system of any of the preceding clauses, wherein said memory device is coupled to said compression sleeve.
Clause 7. The system of any of the preceding clauses, comprising a pressure sensor is configured to communicate pressure in said compression sleeve to said processor.
Clause 8. The system of any of the preceding clauses, said processor is configured to compare said usage of said compression sleeve with a predetermined threshold.
Clause 9. The system of any of the preceding clauses, further comprising at least one physiological sensor coupled to said compression sleeve is configured to communicate at least one physiological signal to said processor.
Clause 10. The system of clause 9, further comprising an alarm in communication with said processor, said processor is configured to trigger said alarm if at least one physiological signal is outside a predetermined range.
Clause 11. The system of claim 9 or 10, further comprising an electrical stimulation device operably connected to said processor.
Clause 12. The system of any of the preceding clauses, wherein said memory device is configured to record the frequency of compressive therapy provided by said compression sleeve.
Clause 13. The system of any of the preceding clauses, wherein said memory device is configured to record the amplitude of compression applied by said compression sleeve. Clause 14. The system of any of the preceding clauses, wherein said memory device is configured to record the duration of application of compressive therapy by said compression sleeve.
Clause 15. The system of any of the preceding clauses, wherein said fluid supply is mounted on said person support apparatus.
Clause 16. The system of any of the preceding clauses, wherein said processor is configured to control at least one function of said person support apparatus.
Clause 17. The system of any of any of the preceding clauses, further comprising a display unit mounted on said person support apparatus, wherein said display unit is configured to display said duration of inflation of said compression sleeve.
Clause 18. A system to monitor compliance of a compression sleeve usage comprising:
   a memory device configured to record usage of a compression sleeve;
   a processor configured to compare said usage with a predetermined threshold, said processor is configured to communicate with a controller of a person support apparatus. Clause 19. The system of clause 18, further comprising a communication network in communication with said processor, said processor is configured to communicate said usage of said compression sleeve by way of said communication network.
Clause 20. The system of clause 18 or 19, further comprising at least one physiological sensor is configured to measure at least one physiological signal and communicate said physiological signal to said processor.
Clause 21. The system of clause 18, 19 or 20, wherein said processor is configured to determine said usage based up on the frequency of compressive therapy provided by said compression sleeve.
Clause 22. The system of any of clauses 18 to 21, wherein said processor is configured to determine said usage based up on amplitude of compression applied by said compression sleeve.
Clause 23. The system of any of clauses 18 to 22, wherein said processor is configured to determine said usage based up on duration of application of compressive therapy by said compression sleeve.
Clause 24. The system of any of clauses 18 to 23, further comprising a display unit mounted on said person support apparatus, said display unit in communication with said processor and is configured to display said usage of said compression sleeve.
Clause 25. A method to monitor compliance of a compression sleeve usage comprising:
   determining usage of a compression sleeve;
   comparing usage of a compression sleeve with a predetermined threshold;
   determining compliance by comparing usage of a compression sleeve with said predetermined threshold.
Clause 26. The method of clause 25, further comprising the step of monitoring at least one physiological signal from a sensor.
Clause 27. The method of clause 25 or 26, further comprising the step of communicating the usage of said compression sleeve to a healthcare database.
Clause 28. The method of clause 25, 26 or 27, further comprising the step of triggering an alarm if the usage of said compression sleeve is below said predetermined threshold.
Clause 29. The method of any of clauses 25 to 28, further comprising the step of communicating with a processor mounted on a person support apparatus.
Clause 30. A system to monitor compliance of a compression sleeve usage comprising:
   a compression sleeve;
   a first processor mounted on said compression sleeve is configured to determine duration of inflation of said compression sleeve and communicate with a second processor mounted on a person support apparatus.

## Claims

1. A person support apparatus (10) for use with a compression sleeve (20), comprising:
a person support surface;
a controller (24) comprising a processor (26) and memory device (28);
a GUI (48) integral to the person support apparatus; and
at least one physiological sensor (32) configured to measure at least one physiological signal and communicate said physiological signal to said processor (26);
wherein:
the controller (24) and GUI (49) are configured to receive user inputs and commands, and control at least one function of said person support apparatus;
the memory device (28) is configured to record usage of the compression sleeve (20), the usage determined by the processor (26);
the memory device (28), or processor (26), is connected to a communication network (40) in communication with at least one of an Electronic Medical Record (44), a nurse call system (46), and a hospital station (42); and
the memory device (28), or processor (26), is configured to communicate, via the communication network (40), signals received from the or each physiological sensor (32) and the recorded usage of the compression sleeve (20) to at least one of the Electronic Medical Record (44), nurse call system (46), and hospital station (42).

2. The person support apparatus according to Claim 1, wherein the processor is configured to determine usage of the compression sleeve (20) for an individual session based on at least one of duration of application of compressive therapy by said compression sleeve (20), amplitude of compression applied by said compression sleeve (20) and frequency of compressive therapy provided by said compression sleeve (20).

3. The person support apparatus according to Claim 1 or 2, further comprising an alarm in communication with said processor (26), the processor (26) configured to trigger the alarm if at least one physiological signal is outside a predetermined range.

4. The person support apparatus according to Claim 3, the processor (26) being configured to trigger the alarm if the usage of said compression sleeve (20) is below a predetermined threshold.

5. The person support apparatus according to any of Claims 1 to 4, further comprising a fluid supply (22) operable to inflate the compression sleeve (20).

6. The person support apparatus according to Claim 5, further comprising a compression sleeve (20) coupled to the fluid supply (22).

7. The person support apparatus according to Claim 5 or 6, wherein the processor (26) is in communication with the fluid supply (22) and is configured to provide a control signal to the fluid supply (22) to inflate or deflate the compression sleeve (20).

8. The person support apparatus according to Claim 7, wherein the processor (26) is configured to communicate with a pressure sensor (34) mounted on the compression sleeve (20) to allow measurement of the pressure inside the inflated compression sleeve (20).

9. The person support apparatus according to Claim 8, wherein the memory device (28) is configured to record parameters related to the use of the compression sleeve (20) for sequential compression therapy.

10. The person support apparatus according to any of claims 6 to 8, wherein the compression sleeve (20) comprises an electrical stimulation device (36) mounted thereto, the memory device configured to record usage of the electrical stimulation device (36).

11. The person support apparatus according to any of the preceding claims, wherein the GUI is configured to display the signals received by the processor (26) from the or each physiological sensor (32) and the recorded usage of the compression sleeve (20).

12. The person support apparatus according to any of the preceding claims, wherein the at least one physiological sensor is configured to output an electrical signal indicative of at least one of the physiological parameters: temperature; blood pressure; heart rate; and electrical activity.
